# EUROPEAN PATENT APPLICATION

(11) **EP 4 736 847 A1**
(43) Date of publication of application: **06.05.2026**
(21) Application number: 24209973.7
(22) Date of filing: 30.10.2024
(51) Int. Cl.: A61K 9/50, A61K 31/05, A61K 31/197, A61K 31/315, A61K 31/385, A61K 31/4172, A61K 31/44, A61K 31/4415, A61K 31/51, A61K 31/519, A61K 31/525, A61K 31/714, A61K 33/30, A61K 36/63, A61K 38/05, A61P 25/00

(54) **CONTROLLED RELEASE GRANULATE**

(71) Applicant: Farmolab S.L., 03540 Alicante (ES); Umbrella Business Group, S.L., 08450 Llinars del Valles Barcelona (ES)
(72) Inventor: Sanchez Rios, Pedro Luis, ALICANTE (ES); Saez Lorente, Francesc Xavier, LLINARS DEL VALLES (BARCELONA) (ES)
(74) Representative: Isern Patentes y Marcas S.L.

(57) **Abstract**

The invention relates to a controlled release granulate comprising: a microcrystalline cellulose core; a first coating comprising vitamin B1, vitamin B2, vitamin B3, vitamin B5, vitamin B6, vitamin B9, vitamin B12, a source of Zn, R-lipoic acid, or an acceptable salt or racemate, L-carnosine or an acceptable salt, olive extract as hydroxytyrosol source, and at least one antioxidant, and a second coating deposited upon the first coating comprising a modified cellulose and/or an hydroalcoholic polymer. Also relates to a process to obtain such controlled release granulate and its use in a method to treat or prevent pain.

## Description

### TECHNICAL FIELD

The present invention generally relates to the food supplements or pharmaceuticals specialties compositions. More particularly, the invention relates to a new formula of controlled release about one composition for the treatment of pain related to nervous system.

### BACKGROUND

In recent years, there has been growing interest in exploring alternative and complementary approaches to managing pain, particularly chronic pain conditions. One area of research that has shown promise is the use of vitamins from the B group, collectively known as B-complex vitamins, for pain management.

The B-complex vitamins, including thiamine (B1), riboflavin (B2), niacin (B3), pantothenic acid (B5), pyridoxine (B6), biotin (B7 or B8 or H), folate or folic acid (B9) or equivalent substances, and cobalamin (B12), play crucial roles in various physiological processes, including energy metabolism, nerve function, and neurotransmitter synthesis. Deficiencies in these vitamins have been linked to neurological disorders and neuropathic pain conditions.

Research studies have indicated that supplementation with B-complex vitamins can have analgesic effects and may help alleviate pain associated with various conditions such as diabetic neuropathy, fibromyalgia, and migraine headaches. Thiamine, for example, has been shown to modulate pain perception and reduce neuropathic pain symptoms by enhancing nerve function and reducing oxidative stress.

Furthermore, B-complex vitamins are generally considered safe and well-tolerated, with few reported adverse effects when used within recommended doses. This makes them an attractive option for pain management, particularly for individuals who may prefer natural or non-pharmacological approaches to pain relief.

Zinc supplements are commonly used to address zinc deficiencies. As an essential mineral, zinc is involved in numerous physiological processes throughout the body. One of its primary functions is to support the immune system, helping to fend off infections and promote faster recovery from illnesses. Additionally, zinc is necessary for proper growth and development, especially during childhood, adolescence, and pregnancy. It aids in wound healing by supporting tissue repair and collagen synthesis. Zinc also contributes to maintaining healthy skin, hair, and nails. Furthermore, zinc is involved in metabolism, including carbohydrate, protein, and lipid metabolism, and it acts as an antioxidant, protecting cells from oxidative damage.

Alpha-lipoic acid (ALA) is a natural compound renowned for its potent antioxidant properties, helping to combat oxidative stress and protect cells from damage. Its role in improving glucose metabolism and insulin sensitivity makes it beneficial for individuals with diabetes, while also showing promise in supporting nerve health and reducing inflammation. ALA may also contribute to skin health, liver function, cognitive performance, and weight management. Although further research is needed to fully understand its effects, ALA remains a versatile supplement with potential benefits across various aspects of health.

L-Carnosine, a dipeptide composed of beta-alanine and histidine, serves various roles in the body as a potent antioxidant and anti-aging agent. It is found abundantly in muscle and brain tissues, it helps buffer acidity in cells, thereby protecting them from oxidative damage and supporting overall cellular function. L-carnosine supplementation has been linked to improved exercise performance, muscle recovery, and enhanced cognitive function. Additionally, it may help mitigate the effects of aging on the skin and eyes, acting as a natural anti-glycation agent to prevent the formation of advanced glycation end products (AGEs).

Hydroxytyrosol is a potent natural antioxidant compound primarily found in olives and olive oil. Its antioxidant properties make it valuable for protecting cells from oxidative damage caused by free radicals, thereby potentially reducing the risk of chronic diseases such as cardiovascular disease, cancer, and neurodegenerative disorders. Additionally, hydroxytyrosol has anti-inflammatory effects, which may help alleviate inflammation-related conditions such as arthritis and inflammatory bowel disease. Moreover, hydroxytyrosol has been shown to support cardiovascular health by improving blood lipid profiles, reducing blood pressure, and enhancing endothelial function. Furthermore, it may have neuroprotective effects, potentially supporting cognitive function and reducing the risk of age-related cognitive decline.

The combination of vitamins B-complex, zinc, carnosine, lipoic acid, and hydroxytyrosol are known for its use in methods for treating pain. However, traditional immediate-release formulations of vitamins B-complex, zinc, carnosine, lipoic acids, and hydroxytyrosol may result in rapid absorption and subsequent rapid elimination from the body, leading to fluctuations in blood levels and potentially diminishing effects. Thus, there is a need for controlled release formulations that offer a controlled and prolonged release of vitamins, ensuring controlled effects over an extended period, thus providing more consistent pain relief.

Additionally, such compositions may have minimized side effects. Rapid fluctuations in blood levels of vitamin 8 compounds can sometimes contribute to adverse effects such as gastrointestinal upset or allergic reactions. Controlled release formulations release the active ingredients gradually, minimizing peak plasma concentrations and reducing the likelihood of side effects.

Some of the mentioned actives exhibit poor oral bioavailability due to factors such as limited solubility or rapid metabolism. Controlled release formulations can optimize the absorption and bioavailability of these compounds by prolonging their residence time in the gastrointestinal tract and facilitating efficient absorption.

Overall, controlled release compositions comprising these actives may represent a significant advancement in the field of pain management, offering improved efficacy, and sustained contribution of actives, patient compliance, safety, and flexibility in dosing regimens. Therefore, there is a need to provide a composition that addresses the drawbacks of the actual compositions and provide long-lasting and consistent relief for individuals suffering from chronic pain conditions.

### SUMMARY OF THE INVENTION

The first aspect of the invention is related to a controlled release granulate comprising:
- a microcrystalline cellulose core;
   a first coating comprising vitamin B1, vitamin B2, vitamin B3, vitamin B5, vitamin B6, vitamin B9, vitamin B12, a source of Zn, R-lipoic acid, or an acceptable salt or racemate, L-carnosine or an acceptable salt, olive extract as hydroxytyrosol source, and at least one antioxidant;
- a second coating deposited upon the first coating comprising a modified cellulose and/or an hydroalcoholic polymer.

The controlled release granulate formulation offers a comprehensive blend of essential vitamins, minerals, and antioxidants, designed to provide controlled release and optimal bioavailability for enhanced health benefits.

In a second aspect, the invention relates to a process of manufacturing the Controlled Release Granulate of the first aspect of the invention. The manufacturing process comprise the following steps:
i.) providing microcrystalline cellulose core;
ii.) weighing and mixing the active agents and the at least one antioxidant with a binder solution;
iii.) spraying the step i). cores with the solution of step ii);
iv.) drying the coated particles of step iii);
v.) spraying a solution comprising modified celluloses, hydroalcoholic polymers and/or an acceptable food dye;
vi.) drying the coated particles of step v); and
vii.) optionally incorporating an anticaking agent to the granule mixture obtained in the previous step.

The third aspect of the invention relates to a unit dose comprising the Controlled Release Granulated or Spherization of the first aspect. In some embodiments, the unitary dose is a capsule, a tablet, or a sachet.

In a fourth aspect, the invention relates to the use of the controlled release granulate of the first aspect or the unit dose of the third aspect in a method of treating or preventing pain in a subject, said method comprising the oral administration of the controlled release granulate of the first aspect or the unit dose of the third aspect.

### BRIEF DESCRIPTION OF THE FIGURE

FIG. 1 shows transparent 00 hard capsules comprising the controlled release granulate prepared according to Example 2.
FIG. 2 depicts the controlled release dissolution profile of capsules comprising the granulate of Example 3 for the main active, lipoic acid, in simulated gastrointestinal conditions. The dissolution test is performed as explained in the examples.

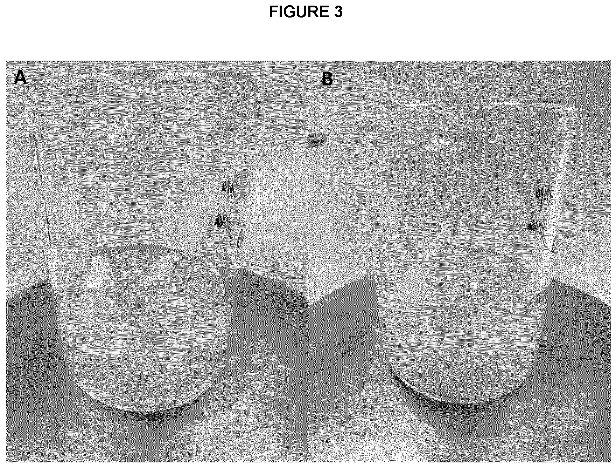

FIG. 3 shows (A) the capsule comprising the granulate at the initial stage (t=0) of the dissolution tests and (B) the dissolution test when the capsules are dissolved (around t=0.2) but the granulate remains.

### DETAILED DESCRIPTION

The first aspect of the invention relates a controlled release granulate comprising:
- a microcrystalline cellulose core;
- a first coating comprising vitamin B1, vitamin B2, vitamin B3, vitamin B5, vitamin B6, vitamin B9, vitamin B12, a source of Zn, R-lipoic acid, or an acceptable salt or racemate, L-carnosine or an acceptable salt, olive extract as hydroxytyrosol source, and at least one antioxidant;
- a second coating deposited upon the first coating comprising a modified cellulose and/or an hydroalcoholic polymer.

While each of these compounds is known for its antioxidant properties individually, among other beneficial effects, when combined in this formulation, they synergistically enhance each other's effects, particularly when the actives are released in a controlled fashion throughout the entire gastrointestinal tract.

The terms "granulate" and "spherization" are used interchangeably and, as used herein, refer to a composition or mixture that has been transformed from a powder into larger particles, referred to as granules, through various methods known in the art.

The terms "core" a granulate refers to the central component or nucleus around which other ingredients or layers are added

In a preferred embodiment, the microcrystalline cellulose core has an average particle size between 100 and 1000 microns, preferably an average particle size between 700 and 1000 microns.

The term "vitamin B1" refer to thiamine or an acceptable salt suitable for human consumption. The term "vitamin B2" refer to riboflavin or an acceptable salt suitable for human consumption. The term "vitamin B3" refer to niacin or an acceptable salt suitable for human consumption. The term "vitamin B5" refer to pantothenic acid or an acceptable salt suitable for human consumption. The term "vitamin B6" is to be understood as pyridoxine or an acceptable salt suitable for human consumption. The term "vitamin B9" is to be understood as folic acid and acceptable salt or suitable for human consumption. The term "vitamin B12" is to be understood as cyanocobalamin or an acceptable salt suitable for human consumption.

In some embodiments, the controlled release granulate comprises:
- between 0.12 and 0.26 wt% of vitamin B1;
- between 0.1 and 0.25 wt% of vitamin B2;
- between 1 and 2.5 wt% of vitamin B3;
- between 0.4 and 0.9 wt% of vitamin B5;
- between 0.15 and 0.35 wt% of vitamin B6;
- between 0.01 and 0.03 wt% of vitamin B9;
- between 0.00005 a 0.002 wt% of vitamin B12;
- between 1 and 13 wt% of a zinc source;
- between 10 and 22 wt% of R-lipoic acid equivalent;
- between 0.6 and 2.25 wt% of olive extract as hydroxytyrosol source;
- between 5 and 9 wt% of a L-carnosine; and
- between 0.1 and 1 wt% of an antioxidant.

Unless specified otherwise, all percentages refer to weight percentages (wt%) relative to the total amount of granulate.

In a preferred embodiment, the controlled release granulate comprises an amount of vitamin B1 between 0.145 and 0.210 wt%; an amount of vitamin B2 between 0.125 and 0.200 wt%; an amount of vitamin B3 between 1.390 and 2.100 wt%; an amount of vitamin B5 between 0.510 and 0.750 wt%; an amount of vitamin B6 between 0.190 and 0.300 wt%; an amount of vitamin B9 is between 0.015 and 0.025 wt%; an amount of vitamin B12 between 0.000077 and 0.00120 wt%; an amount of a zinc source between 1.10 and 3.22 wt%; an amount of lipoic acid or acceptable salts between 13.00 and 18.60 wt%; an amount of L-carnosine or acceptable salts between 6.50 and 7.70 wt%, and an amount of olive extract as hydroxytyrosol source between 0.6 and 2.25 wt%.

In some embodiments, the controlled release granulates' source of zinc is an organic or inorganic salt. In a preferred embodiment the source of zinc is selected from zinc gluconate, zinc pidolate, zinc picolinate, zinc citrate, zinc sulphate, zinc orotate, zinc chloride, zinc acetate, zinc lactate, zinc oxide, zinc bisglycinate or mixtures thereof. In the preferred embodiment of the controlled release granulate the source of zinc is zinc sulphate, most preferably zinc sulphate monohydrate.

In some embodiments, the granulate comprises R-lipoic acid in a form selected from alpha R-lipoic acid or sodium R-lipoate. In the preferred embodiment the granulate comprise R-lipoic acid as a racemate or racemic mixture of R-(+)-lipoic acid and S-(-)-lipoic acid.

In some embodiments, the granulate comprises L-carnosine in the form of L-carnosine, N-acetyl Carnosine or as a carnosine complex.

In some embodiments, the granulate comprise at least 0.6 wt% of olive extract as hydroxytyrosol source, preferably comprise between 0.6 and 2.25 wt% of olive extract as hydroxytyrosol source in respect the controlled release granulate. The hydroxytyrosol amount in commercially available olive extract may range between from 2% to 40 wt% in respect to the olive extract.

In another embodiment, the antioxidant is selected from L-ascorbyl palmitate, alpha-tocopherol or Vitamin E derivates, L-Ascorbic Acid or any of its salts, citric acid or any of its salts, butylated hydroxyanisole (BHA), gallates such as propyl gallate, octyl gallate, and dodecyl gallate, butylated hydroxytoluene (BHT) or mixtures thereof. In the preferred embodiments, the antioxidant is L-ascorbyl palmitate, and it is present from 0.1 to 1 wt% in respect the controlled release granulate.

The term "antioxidant" refers to a substance included in pharmaceutical compositions that inhibits oxidation or reactions promoted by oxygen, peroxides, or free radicals, thereby protecting the active ingredients and ensuring the stability and efficacy of the formulation.

In a preferred embodiment, the second coating comprises hydroxypropyl methylcellulose, methacrylic acid copolymers, hypromellose phthalate, cellulose acetate phthalate, polyvinyl acetate phthalate or hydroxypropyl methylcellulose acetate succinate.

In a most preferred embodiment, the controlled release granulate comprises:
- between 0.12 and 0.26 wt% of vitamin B1;
- between 0.1 and 0.25 wt% of vitamin B2;
- between 1 and 2.5 wt% of vitamin B3;
- between 0.4 and 0.9 wt% of vitamin B5;
- between 0.15 and 0.35 wt% of vitamin B6;
- between 0.01 and 0.03 wt% of vitamin B9;
- between 0.00005 a 0.002 wt% of vitamin B12;
- between 1 and 13 wt% of a zinc source;
- between 10 and 22 wt% of R-lipoic acid equivalent;
- between 0.6 and 2.25 wt% of an olive extract as hydroxytyrosol source;
- between 5 and 9 wt% of L-carnosine; and
- between 0.1 and 1 wt% of an antioxidant.

In another embodiment of the first aspect the controlled release granule, the granule has an average particle size between 100 and 1000 microns measured with a sieve method and/or vernier caliper. In a preferred embodiment, the granulate has ana average particle size between 700 and 1000 microns measured with a sieve and/or vernier caliper.

In another embodiment of the first aspect, the water content of the controlled release granulate is between 5% and 21% w/w in respect of the total weight of the initial granulate when measured by weight loss with moisture analyser, more preferably the water content of the spherization is between 0.1% and 5% w/w in respect of the total weight of the final spherization when measured by weight loss with moisture analyser.

In some embodiments, the controlled release granulate maintains its structural integrity and remains undissolved for a minimum duration of 2 hours, when subjected to an in vitro dissolution simulating gastrointestinal conditions. In some embodiments, the controlled release granulate less than 10 wt% of the total amount of lipoic acid contained in the granulate is released within 8 hours, when subjected to an in vitro dissolution simulating gastrointestinal conditions.

In some embodiments, the controlled release granulate maintains its structural integrity and remains undissolved for a minimum duration of 2 hours, when subjected to an in vitro dissolution test using 50mL of 0.1 N hydrochloric acid, 20000U/MI pepsine at pH 3 as a dissolution medium at 450 rpm and 37°C. In some embodiments, the controlled release granulate less than 10 wt% of the total amount of lipoic acid contained in the granulate is released within 8 hours, when subjected to an in vitro dissolution test using 50 mL of 0.1 N hydrochloric acid, 20000U/MI pepsin at pH 3 as a dissolution medium at 450 rpm during 2h at 37°C and then incorporate to the dissolution media 50 mL of 1600U/mL pancreatin. 160Mm bovine bile at pH 7 and maintain the resulting solution at 37°C during 6 additional hours.

The term "active agent" or "active compound" refers to a therapeutically active compound and pharmaceutically acceptable salts, hydrates and solvates of the compound. It specifically refers the combination of vitamin B1, vitamin B2, vitamin B3, vitamin B5, vitamin B6, vitamin B9, vitamin B12, Zn, R-lipoic acid, L-carnosine- and hydroxytyrosol.

In a further embodiment, the controlled release granulate does not comprise additional active agents other than vitamin B1, vitamin B2, vitamin B3, vitamin B5, vitamin B6, vitamin B9, vitamin B12, a source of Zn, R-lipoic acid or an acceptable salt or racemate, L-carnosine or an acceptable salt, and olive extract as a source of hydroxytyrosol.

The second aspect of the invention relates to a manufacturing process that comprise the following steps:
i.) providing microcrystalline cellulose core;
ii.) weighing and mixing the active agents and the at least one antioxidant with a binder solution;
iii.) spraying the step i). cores with the solution of step ii);
iv.) drying the coated particles of step iii);
v.) spraying a solution comprising modified celluloses, hydroalcoholic polymers and/or an acceptable food dye;
vi.) drying the coated particles of step v); and
vii.) optionally incorporating an anticaking agent to the granule mixture obtained in the previous step.

In some embodiments, the binder solution in step ii) is a solution of hydroxypropyl methylcellulose or hypromellose. In some preferred embodiments, the binder solution in step ii) is an aqueous or hydroalcoholic solution of hydroxypropyl methylcellulose or hypromellose.

The dye and anticaking agent may be selected from many of the options known by the skilled persons in this field. In some embodiments, the granulate mixture comprises an anticaking agent. Preferably, the anticaking agent is silicon oxide.

In the preferred embodiments, step iv) and vi) are carried out under controlled conditions, preferably maintaining the temperature between 10 and 30 °C.

In some embodiments, the resulting controlled release granule has an average particle size between 100 and 1000 microns measured with a sieve method and/or vernier caliper. In a preferred embodiment, the granulate has ana average particle size between 700 and 1000 microns measured with a sieve and/or vernier caliper.

Third aspect of the invention refers to a single unit dosage comprising the controlled release granulate of the first aspect. In some embodiments the unitary dose is an oral administration form.

As used herein, the term "unit dose", "unit dosage" or "unitary dose" refers to a physically discrete unit that contains a predetermined quantity of active agent calculated to produce a desired therapeutic effect. In some embodiments, the single unit dosage is in form of tablet, capsule, or sachet adapted from usual systems of packaging.

A further embodiment of the present aspect relates to a hard capsule comprising between 500 and 750 mg of a composition comprising a controlled release granulate comprising the active agents, preferably the capsule comprises between 600 and 700 mg of the controlled release granulate.

In some embodiments the granulate or the composition according to the previous aspects is used as a food supplement. The term "food supplement", also known as a dietary supplement, is a product intended to supplement the diet and provide additional concentrate nutrients such as vitamins, minerals, amino acids, fatty acids, or other substances that may be missing or insufficient in a person's regular diet and under an established daily dose. In some embodiments, the granulate or the composition according to the previous aspects is used in the treatment of pain such as radicular pain, pain associated with radiculopathy, neuropathic or sciatic pain.

In a fourth aspect, the invention relates to the use of the controlled release granulate of the first aspect or the unit dose of the third aspect in a method of treating or preventing pain in a subject, said method comprising the oral administration of the controlled release granulate of the first aspect or the unit dose of the third aspect.

In some embodiments, the granulate or the composition may be used in the treatment of pain such as radicular pain, pain associated with radiculopathy, neuropathic or sciatic pain, by administering a daily dosage of 150 to 750 mg of active substances administered in one to three doses. In certain embodiments, the single unit dose is administered at least once daily, ideally between one and three single unit doses per day.

### EXAMPLES

### 1. Compositions of the invention

| | **Example 1** | **Example 2** | **Example 3** |
|---|---|---|---|
| **Component** | **Quantity (wt%)** | | |
| Microcrystalline Cellulose | 28.3 | 28.1 | 27.8 |
| Vitamin B1 (Thiamine HCl) | 0.12 | 0.145 | 0.195 |
| Vitamin B12 (Cobalamin) 0.1 wt% | 0.05 | 0.08 | 0.115 |
| Vitamin B2 (Riboflavin) | 0.1 | 0.125 | 0.17 |
| Vitamin B3 (Nicotinamide) | 1 | 1.39 | 1.66 |
| Vitamin B5 (Calcium D-Pantothenate) | 0.4 | 0.51 | 0.69 |
| Vitamin B6 (Pyridoxine HCl) | 0.15 | 0.19 | 0.26 |
| Vitamin B9 (Folic Acid) | 0.01 | 0.015 | 0.02 |
| ZnSO₄ H₂O | 1 | 2.6 | 3.21 |
| *R*-alpha-Lipoic Acid | 10 | 13 | 15.54 |
| Hydroxypropylmethylcellulose | 16 | 15.8 | 15.04 |
| L-Carnosine | 5 | 6.5 | 7.69 |
| L-Ascorbyl Palmitate | 0.3 | 0.36 | 0.37 |
| Olive fruit dry extract 9.9% Hydroxytyrosol | 5 | 6.5 | 7.7 |
| SiO₂ low density | 3 | 3.1 | 3.12 |
| Water | q.s | q.s | q.s |

| | **Example 4** | **Example 5** |
|---|---|---|
| **Component** | **Quantity (wt%)** | |
| Microcrystalline cellulose | 27.5 | 28 |
| Vitamin B1 (Thiamine HCl) | 0.21 | 0.25 |
| Vitamin B12 (Cobalamin) 0.1 wt% | 0.12 | 0.15 |
| Vitamin B2 (Riboflavin) | 0.20 | 0.25 |
| Vitamin B3 (Nicotinamide) | 2.10 | 2.5 |
| Vitamin B5 (Calcium D-Pantothenate) | 0.75 | 0.8 |
| Vitamin B6 (Pyridoxine HCl) | 0.30 | 0.35 |
| Vitamin B9 (Folic Acid) | 0.03 | 0.03 |
| ZnSO₄ H₂O | 3.20 | 5 |
| R-alpha-Lipoic Acid | 15.60 | 17 |
| Hydroxypropylmethylcellulose | 14.40 | 15 |
| L-Carnosine | 7.70 | 8 |
| L-Ascorbyl Palmitate | 0.37 | 0.3 |
| Olive Fruit Dry Extract 9.9% Hydroxytyrosol | 7.80 | 8 |
| SiO₂ low density | 3.40 | 3.5 |
| Water | q.s | q.s |

### 2. Granulation process

The examples' compositions were prepared according to the detailed process outlined below:
i.) providing microcrystalline cellulose cores;
ii.) weighing and mixing the active agents and L-Ascorbyl Palmitate as antioxidant with a HPMC solution;
iii.) spraying the step i). cores with the solution of step ii);
iv.) drying the coated particles of step iii) at 30 °C;
v.) spraying a solution comprising hypromellose and an acceptable food dye;
vi.) drying the coated particles of step v) at 30 °C; and
vii.) incorporating silicon oxide as anticaking agent to the granule mixture obtained in the previous step.

The Controlled Release Granules resulting from the manufacturing process, can be conveniently packed into single unit dosages such as capsules, tablets, or sachets using well-established methods within the field.

### 3. Dissolution test in simulated gastrointestinal conditions

The correct effect and its sustained release over time of the granulate have been simulated "in vitro." The dissolution profile of the granules, when supplied in a 00 capsule, was evaluated under conditions simulating the human gastric (acid & enzyme) and intestinal (basic & enzyme) systems, focusing on R-Lipoic acid, as representative of the liberation of the actives, since it is the most sensitive, delicate, and main mechanism of therapeutic action.

The dissolution media employed in the dissolutions profiles is labelled "gastric" and "intestinal" and each seeks to simulate the conditions of a specific part of the gastrointestinal system. The gastric simulating media consisted 50 mL of 0.1 N hydrochloric acid, 20000U/MI pepsin at pH 3 stirred at 450 rpm and maintained at 37°C. The intestinal simulated media consisted in a mix in a 1:1 ratio of gastric media and a dissolution of 1600U/mL pancreatin, 160Mm bovine bile at pH 7 stirred at 450 rpm and maintained at 37°C.

A release curve of the lipoic acid of capsules containing 650 mg of the granulate of example 3 was conducted. To achieve this, 4 tests (each containing two capsules) are set up in parallel, each corresponding to a different release time (gastric study (2h); intestinal studies (4h, 6h, 8h)).

All the dissolution tests described began under simulated gastric conditions for 2 hours. Subsequently, the media was adjusted to simulate intestinal conditions as previously explained. At the end of the dissolution test (8h), the partially undissolved granules were identifiable.

The determination of lipoic acid was conducted according to the "Alpha lipoic acid capsules 2024 USP monograph" of the USP Pharmacopeia 2024, prior centrifugation of the sample.

| **Dissolution Media** | **Time (cumulative) (h)** | **Number of capsules** | **Total lipoic liberated per capsule (650 mg)** | **Total lipoic liberated per capsule (%)** |
|---|---|---|---|---|
| Gastric | 2 | 2 | 40.20 | 6.18 |
| Intestinal | 4 | 2 | 56.20 | 8.64 |
| Intestinal | 6 | 2 | 57.1 | 8.78 |
| Intestinal | 8 | 2 | 60.1 | 9.25 |

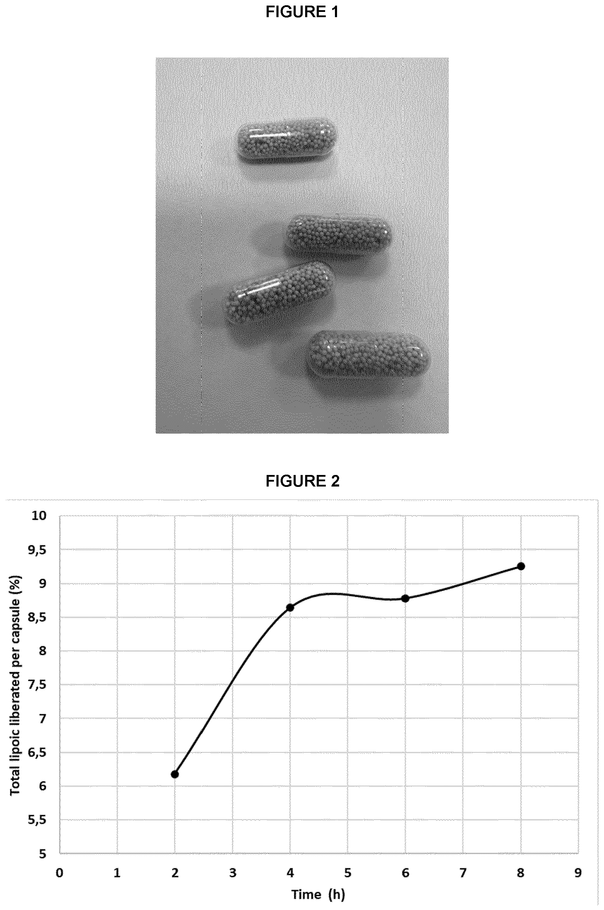

As shown in the table above and in Figure 2, the dissolution profile of the controlled release granules demonstrates that the primary component of the composition is released in a controlled manner throughout all simulated stages for at least 8 hours. Based on this, we can conclude that all active ingredients within the granulate exhibit-controlled release.

## Claims

1. A controlled release granulate comprising:
- a microcrystalline cellulose core;
- a first coating comprising vitamin B1, vitamin B2, vitamin B3, vitamin B5, vitamin B6, vitamin B9, vitamin B12, a source of Zn, R-lipoic acid, or an acceptable salt or racemate, L-carnosine or an acceptable salt, olive extract as hydroxytyrosol source, and at least one antioxidant;
- a second coating deposited upon the first coating comprising a modified cellulose and/or an hydroalcoholic polymer.

2. The controlled release granulate according to the previous claim in which a less than 10 wt% of the total amount of lipoic acid contained in the granulate is released within 8 hours, when subjected to an in vitro dissolution test using 50 mL of 0.1 N hydrochloric acid, 20000U/MI pepsin at pH 3 as a dissolution medium at 450 rpm during 2h at 37°C and then incorporate to the dissolution media 50 mL of 1600U/mL pancreatin. 160Mm bovine bilis at pH 7 and maintain the resulting solution at 37°C during 6 additional hours.

3. The controlled release granulate according to any of the previous claims, wherein the microcrystalline cellulose core has an average particle size between 100 and 1000 microns, preferably the average particle size is between 700 and 1000 microns.

4. The controlled release granulate according to any of the previous claims, wherein
- between 0.12 and 0.26 wt% of vitamin B1;
- between 0.1 and 0.25 wt% of vitamin B2;
- between 1 and 2.5 wt% of vitamin B3;
- between 0.4 and 0.9 wt% of vitamin B5;
- between 0.15 and 0.35 wt% of vitamin B6;
- between 0.01 and 0.03 wt% of vitamin B9;
- between 0.00005 a 0.002 wt% of vitamin B12;
- between 1 and 13 wt% of a zinc source;
- between 10 and 22 wt% of R-lipoic acid equivalent;
- between 0.6 and 2.25 wt% of an olive extract as hydroxytyrosol source;
- between 5 and 9 wt% of L-carnosine; and
- between 0.1 and 1 wt% of an antioxidant.

5. The controlled release granulate according to any of the preceding claims, wherein the second coating comprises hydroxypropyl methylcellulose, methacrylic acid copolymers, hypromellose phthalate, cellulose acetate phthalate, polyvinyl acetate phthalate or hydroxypropyl methylcellulose acetate succinate.

6. The controlled release granulate according to any of the preceding claims, wherein the granulate further comprises an anticaking agent, preferably the anticaking agent is silicon oxide.

7. The controlled release granulate according to any of the preceding claims, wherein the antioxidant is selected from L-ascorbyl palmitate, L-ascorbic acid or any of its salts, alpha-tocopherol, citric acid or any of its salts, butylated hydroxyanisole (BHA), gallates, butylated hydroxytoluene (BHT) or mixtures thereof, preferably the antioxidant is L-ascorbyl palmitate.

8. A process for the preparation of a controlled release granulate according to any of the preceding claims, comprising the following steps:
i.) providing microcrystalline cellulose core;
ii.) weighing and mixing the active agents and the at least one antioxidant with a binder solution;
iii.) spraying the step i). cores with the solution of step ii);
iv.) drying the coated particles of step iii);
v.) spraying a solution comprising modified celluloses, hydroalcoholic polymers and/or an acceptable food dye;
vi.) drying the coated particles of step v); and
vii.) optionally incorporating an anticaking agent to the granule mixture obtained in the previous step.

9. The process for the preparation of a granulate according to the preceding claim, wherein the binder solution is an aqueous or hydroalcoholic solution of hydroxypropyl methylcellulose or hypromellose.

10. A composition comprising the controlled release granulate according to any of the preceding claims 1-7.

11. The composition according to preceding claim, wherein the composition is an oral composition, preferably in form of capsule, sachet, or tablet, most preferably in form of capsule.

12. The composition according to the preceding claim, wherein the composition comprises an amount of active agents per unit between 150 and 250 mg.

13. The granulate according to the preceding claims 1 to 7 or the composition according to any one of claims 8-9, for use in the treatment of pain such as radicular pain, pain associated with radiculopathy, neuropathic or sciatic pain.
